(19) Europäisches Patentamt
European Patent Office
Office européen des brevets

(11) Publication number: 0 361 566 A1

# (12) EUROPEAN PATENT APPLICATION

(21) Application number: 89202198.1

(22) Date of filing: 31.08.89

(51) Int. Cl.5: C07D 471/04 , A61K 31/44 , //(C07D471/04,221:00,209:00)

(30) Priority: 28.09.88 IT 6787188

(43) Date of publication of application: 04.04.90 Bulletin 90/14

(84) Designated Contracting States: AT BE CH DE FR GB IT LI NL SE

(71) Applicant: FARMIGEA S.P.A.
Via Carmignani, 2
I-56127 Pisa(IT)

(72) Inventor: Bianchini, Pietro
Via Del Sagittario, 58
I-41100 Modena(IT)
Inventor: Da Settimo, Federico
Via Santa Cecilia, 2
I-56100 Pisa(IT)

(74) Representative: Lotti, Giorgio
c/o Ing. Barzanò & Zanardo Milano S.p.A. Via Cernaia 20
I-10122 Torino(IT)

## (54) Derivatives of 7-amino 2-dialkylaminoalkyl-1H-pyrrol[3.4-c] pyridine-1.3(2H)-dione with effect against cataract.

(57) The invention refers to new efficacious products for the prevention and therapy of cataract, derivatives of 7-amino-2-dialkylaminoalkyl-1H-pyrrol [3.4-c] pyridine-1.3(2H)-dione of general formula, I

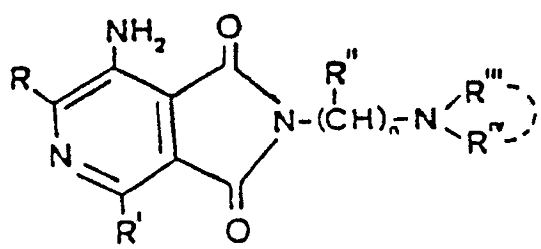

I

where R represents an atom of hydrogen, a methyl or ethyl group, R′ an atom of hydrogen or of chlorine, the chain between the two atoms of nitrogen of the lateral chain consists of 2 or 3 atoms of carbon (n = 2.3) and it may be linear or branched with small groups of alkyls (R″ = hydrogen, methyl or ethyl); groups R‴ and $R^{IV}$ are $CH_3$ $C_2H_5$,

$$-CH\begin{matrix}CH_3\\CH_3\end{matrix}$$

or they are part of a ring such as the one of pyrrolidine, of piperidine, of N-methylpiperazine, of β-hydroxyethylpiperazine or of morpholine.

EP 0 361 566 A1

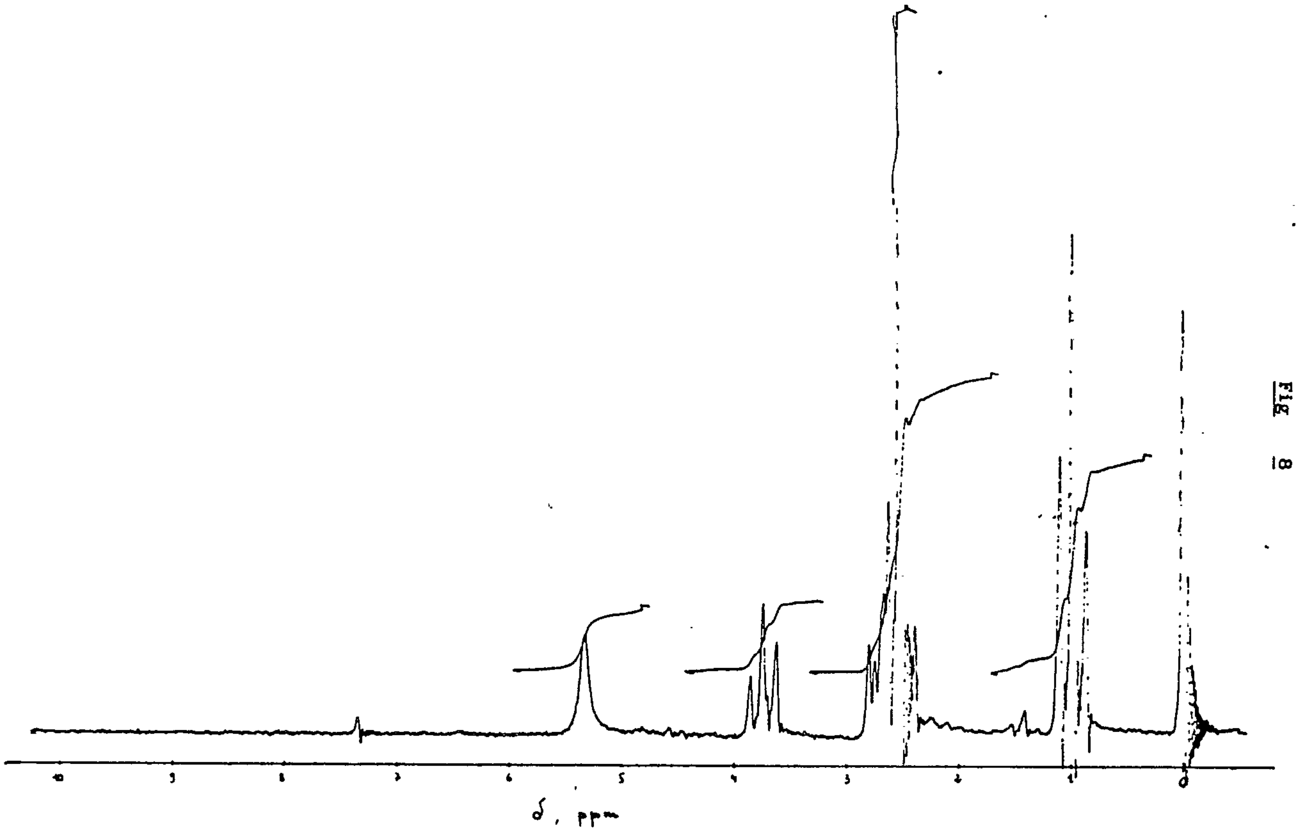


Fig 8

# DERIVATIVES OF 7-AMINO-2-DIALKYLAMINOALKYL-1H-PYRROL [3.4-c] PYRIDINE-1.3(2H)-DIONE WITH EFFECT AGAINST CATARACT

The invention refers to new efficacious products for the prevention and therapy of cataract, derivatives of 7-amino-2-dialkylaminoalkyl-1H-pyrrol [3.4-c] pyridine-1.3(2H)-dione of general formula, I

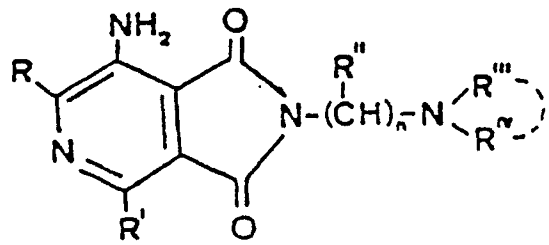

I

where R represents an atom of hydrogen, a methyl or ethyl group, R′ an atom of hydrogen or of chlorine, the chain between the two atoms of nitrogen of the lateral chain consists of 2 or 3 atoms of carbon (n = 2,3) and it may be linear or branched with (small groups of alkyls (R″ = hydrogen, methyl or ethyl); groups R‴ and $R^{IV}$ are $CH_3$, $C_2H_5$,

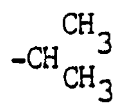

or they are part of a ring such as the one of pyrrolidine, of piperidine, of N-methylpiperazine, of β-hydroxyethylpiperazine or of morpholine, etc.

Hydrochlorates of all products are prepared, and so are the salts of organic acids such as ascorbic acid, glutamic acid, maleic acid, etc.

The products of the invention are obtained with known methods for the preparation technique of imides. The preferred method consists in causing a reaction of pyridine-4.5 dicarboxilic acid properly susbstituted with suitable diamine at melting temperature.

Some illustrative but not limiting examples of the invention are going to be described hereinafter; the attached drawings illustrate the IR or NMR spectrums of the compounds of said examples.

EXAMPLE 1 - Preparation of: 7-amino-dialkylamino alkyl-1H-pyrrol [3.4-c] pyridine-1.3(2H)-dione (I)

0.0055 moles of the suitable 3-aminopyridine-4.5-dicarboxylic acid are added to 0.006 moles of the suitable amine, cooling in ice bath. The reaction mixture is heated for two hours at 170°. In case diysopropylamino-ethylamine is employed, it is heated at 190°.

After cooling the raw product obtained is extracted with benzene, the benzene solution is concentrated to small volume and filtered on alumina column (h = 3-5 cm; ∅ = 1.5 cm) eluting with benzene. A yellow-orange solid substance (is obtained which supplies a yellow crystalline product (I) (yield 30-70%) once crystallized with proper solvent.

(I) is transformed in hydrochlorate by means of hydrochloric ethanol; a crystalline, yellow product, soluble in water is obtained (yield 70/100°) by means of crystallization by methanol-ethyl ether.

EXAMPLE 2 - Preparation of: 7-amino-2-(2-diethylamino ethyl)-1H pyrrol [3.4-c] pyridine-1.3(2H)-dione (R = R′ = R″ = H; R‴ = $R^{IV}$ = $C_2H_5$; n = 2) (Ia)

G 0.810 (0.0044 moles) of 3-aminopyridine-4.5 dicarboxilic acid are added to ml 0.73 (0.0049 moles) of N,N-diethylendiamine, cooling in ice bath; the reaction mixture is heated for two hours at 170°. After cooling the solid residual is extracted with benzene; the benzene solution is concentrated to small volume at

reduced pressure, then filtered on alumina column (h = 3 cm; ∅ = 1.5 cm) eluting with benzene. A yellow solid substance is obtained which supplies 0.670 g (yield 58%) of (Ia) yellow crystalline product, m.p. 63-64° once crystallized by gasoline 60/80°; IR spectrum is illustrated in Fig. 1. (Ia) is transformed in its hydrochlorate by dissolution in methanol and addition of hydrochloric ethanol, obtaining 0.700 g (yield 82%) of yellow crystalline product, soluble in water. An analytical sample is obtained through crystallization by methanol-ethyl ether; m.p. 262-264°; IR spectrum is illustrated in Fig. 2; NMR spectrum is illustrated in Fig. 3.

EXAMPLE 3 - Preparation of: 7-amino-2-diethylamino ethyl)-6-methyl-1H-pyrrol [3.4-c] pyridine-1.3(2H)-dione ($R = CH_3$; $R' = R'' = H$; $R''' = R^{IV} = C_2H_5$; n = 2) (Ic)

G 1.176 (0.0055 moles) of 2-methyl-3-amino-pyridine-4.5-dicarboxilic acid are added to ml 0.9 (0.006 moles) of N,N-diethylendiamine and cooled in ice bath; the reaction mixture is heated for two hours at 170°. After cooling, the raw product obtained is extracted with benzene and the benzene solution is filtered on alumina column (h = 8 cm; ∅-1 cm) eluting with benzene. A residual is obtained which crystallized by oil ether, supplies 0.700 g (yield 46%) of (Ic), yellow crystalline product, m.p. 50-52°; IR spectrum is illustrated in Fig. 4; NMR spectrum is illustrated in Fig. 5.

(Ic) is transformed in its hydrochlorate by dissolution in methanol and by addition of hydrochloric ethanol, obtaining 0.650 (yield 73%) of water soluble yellow crystalline product. An analytical sample is prepared through crystallization by methanol-ethyl ether: yellow crystalline product, m.p. 249-251° (dec.); IR spectrum is illustrated in Fig. 6.

EXAMPLE 4 - Preparation of 7-amino-4-chlorine-2-(2- diethylaminoethyl)-6-methyl-1H-pyrrol [3.4-c] pyridine 1 .3 (2H)-dione ($R = CH_3$, $R' = C1$; $R'' = H$; $R''' = R^{IV} = C_2H_5$; n = 1) (Im)

G 1.150 (0.005 moles) of 3-amino-6-chlorine-2-methylpyridine-4.-5-sdicarboxylic acid are added to ml 0.8 (0.0055 moles) of N,N-diethylendiamine, and cooled in ice bath; the reaction mixture is heated for two hours at 170°. After cooling the reaction solid residual is extracted with benzene. The benzene solution is dry distilled by reduced pressure furnishing a brown solid substance which is dissolved in a little benzene and filtered on alumina column (h = 5 cm; ∅ = 1.5 cm) eluting with benzene. A yellow-orange solid substance is obtained, which supplies 0.750 g (yield 48%) of crystalline yellow product (Im), m.p. 131-132°, when crystallized by gasoline 60-80°; IR spectrum is illustrated in Fig. 7; NMR spectrum is illustrated in Fig. 8.

(Im) is transformed in its hydrochlorate by dissolution in methanol and addition of hydrochloric ethanol, obtaining 0.700 g (yield 75%) of water soluble yellow crystalline product.

An analytical sample is prepared through crystallization by methanol-ethyl ether; yellow crystalline product; m.p. 248-250° = (dec); spectrum IR is illustrated in Fig. 9.

TABLE 1 of the following page indicates data relating to other products:

Analytical data of all new products prepared are in accordance with the calculated values (±0.3%), as well as spectrums I.R. and ′H N M.R.

PHARMACOLOGIC DATA

TEST OF CATARACT PREVENTION ON LIVING BEINGS

Diabetic type cataract

The formation of diabetic type cataract has been induced in Sprague-Dawley male albino rats through normal feeding supplemented with 30% or 50% of galactose. Consideration has been given to cortical cataract of the type illustrated by Creighton M.O., Trevitmick J.R., Cortical cataract formation prevented by vitamin E and glutathione, Exp. Eye Res. 1979, 29, 689-693 and nuclear cataract which arise in galactosemic rats respectively after 4-5 and 10-15 days from the beginning of the diet and involving 70-90% of the animals under control after 21-28 days, end of the experiment, as illustrated respectively by Dvornik D. Simard, Duquesne N., Krami M., Stestanj K., Polyol accumulation in galactosemic and diabetic rats: control

by an aldose reductase inhibitor, Science 1973, 182, 1146-7; by Datiles M.,

TABLE 1

| n° | R | R' | R" | 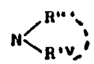 | n | Crystall. solvent | p.f. (°C) | Yield % | Dihydrochlorate crystal. solvent | p.f. (°C) | Yield % |
|---|---|---|---|---|---|---|---|---|---|---|---|
| I a | H | H | H | N $(C_2H_5)_2$ | 2 | gasoline 60-80° | 63-64 | 58 | methanol/ethyl ether | 262-264 | 82 |
| I b | Me | H | H | N $(CH_3)_2$ | 2 | gasoline 60-80° | 121-123 | 50 | methanol/ethyl ether | > 300 | 90 |
| I c | Me | H | H | N $(C_2H_5)_2$ | 2 | oil ether | 50-52 | 46 | methanol/ethyl ether | 249-251(dec) | 73 |
| I d | Me | H | H | N $(i\text{-}C_3H_7)_2$ | 2 | gasoline 60-80° | 158-160 | 36 | methanol | 250-252 | 83 |
| I e | Me | H | H | 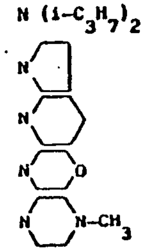 | 2 | gasoline 100-140° | 130-132 | 42 | methanol | > 300 | 93 |
| I f | Me | H | H | | 2 | gasoline 60-80° | 117-119 | 50 | methanol/ethyl ether | > 300 | 51 |
| I g | Me | H | H | | 2 | gasoline 100-140° | 184-186 | 53 | methanol/ethyl ether | 270-272(dec) | 95 |
| I h | Me | H | H | | 2 | gasoline 60-80° | 122-123 | 56 | methanol/ethyl ether | 201-204(dec) | 85 |
| I i | Me | H | H | N $(C_2H_5)_2$ | 3 | gasoline 60-80° | 81-83 | 69 | methanol/ethyl ether | 253-255(dec) | 100 |
| I l | Me | Cl | H | N $(CH_3)_2$ | 2 | gasoline 100-140° | 175-177 | 56 | methanol/ethyl ether | > 300 | 100 |
| I m | Me | Cl | H | N $(C_2H_5)_2$ | 2 | gasoline 60-80° | 131-132 | 48 | methanol/ethyl ether | 248-250(dec | 75 |
| I n | Me | Cl | H | N $(i\text{-}C_3H_7)_2$ | 2 | gasoline 100-140° | 216-216 | 66 | methanol/ethyl ether | 276-278 | 92 |
| I o | Me | Cl | H | 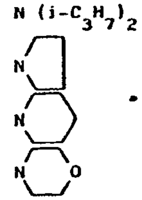 | 2 | gasoline 100-140° | 156-158 | 57 | methanol/ethyl ether | > 300 | 65 |
| I p | Me | Cl | H | | 2 | gasoline 100-140° | 187-189 | 56 | methanol/ethyl ether | 273-275 | 70 |
| I q | Me | Cl | H | | 2 | benzene | 198-200 | 63 | methanol | 244-246(dec | 92 |
| I r | Me | Cl | H | N $(C_2H_5)_2$ | 3 | gasoline 100-140° | 115-117 | 55 | methanol/ethyl ether | 222-225(dec | 60 |

Fukvi H., Kuwabara T., Knoshita J.H., Galactose cataract prevention with Sorbinil, an aldose reductase inhibitor: a light microscopic study, Invest. Ophthalm. Visual Sc. 1982, 22, 174-179 and by Okuda J., Yashima K., Inaqaki K., Miwa I., Effect of an aldose reductase inhibitor 1- [(p-Bromophenyl) Sulfonyl] Hidantoin, on cataract formation and tissue polyol levels in galactosemic rats, Chem. Pharm. Bull. 1985, 33-(7), 2990-5.

Cataracts have been observed either directly or by a fissure lamp.

At the end of the treatment, the inhibition of the cataract formation has been evaluated according to percentage, comparing the number of animals with cataract in the various groups of rats treated with substances having a supposed cataract prevention effect, with the number of animals with cataract in the control group.

The substances prepared as hydrochlorates have been administered daily, the dose being 100 mg/Kg, constantly employing the volume of 0.4 ml/100 g. The animals under control have received the same volume of physiological solution. Some test results are listed hereafter:

Table 2

| DIABETIC TYPE CATARACT | | |
|---|---|---|
| Substances | Dose (mg/Kg/orally) | Inhibition % |
| I a (DS 56) | 100 | 35.17 |
| I c (DS 54-55) | 100 | 36.65 |
| I d (DS 100) | 100 | 49.09 |
| I m (DS 94) | 100 | 27.60 |
| I n (DS 101) | 100 | 42.16 |

Some of the above substances have also been employed in the following test which allows to obtain senile type cataracts in rats as illustrated by Yamauchi T., Komura S., Yaqi K., Serum lipid peroxide levels of albino rats administered naphtalene, Biochemistry International 1986, 13, 1-6.

Senile type cataract

The senile type cataract has been obtained administering daily an oral dose of 0.4-0.5 g/Kg of 10% naphtalene dissolved in peanut oil to Sprague-Dawley albino male rats. Cataracts are formed 6-10 days after beginning of treatment and after 18-28 days involve 100% of the animals employed. Cataracts, which are to be evaluated only by means of fissure lamp, are described by opportune scores.

The substances having a supposed effect against cataract are also administered orally according to modalities and doses already described for the diabetic type cataract. Also in the case of senile type cataract the determination of the inhibition percentage is carried out with reference to the number of animals with cataract; regarding the scores, the tendency of the cataract formation process in the various groups of animals, is described by diagrams.

Some of the substances of the present invention, such as Ic (DS 54-55) and Id (DS 100), have already been tried by this test and have resulted having an inhibiting effect equal to 15-25%. Other substances of the same series, such as Im (DS 94) and In (DS 101) have been prepared as diascorbates for application, in suitable doses in the conjunctival sack of rats and rabbits, in order to evaluate the possibility of a transcorneal passage to anterior aqueous chamber and successive possibility of a direct effect against cataract formation.

## Claims

1) Compounds of general formula (1) where R represents an atom of hydrogen, a methyl or ethyl group;

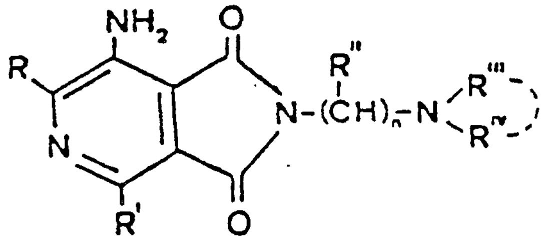

I

R′ an atom of hydrogen or chlorine; (n = 2.3); R″ is hydrogen, methyl or ethyl; groups R‴ and $R^{IV}$ are $CH_3$ $C_2H_5$,

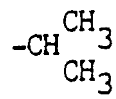

or they are part of a ring such as the one of pyrrolidine, of piperidine, of N-methylpiperazine, of β-hydroxyethylpiperazine or of morpholine.

2) Compounds according to claim 1 wherein they are in the shape of salts pharmaceutically acceptable such as hydrochlorate, ascorbate, glutamate, maleate.

3) 7-amino-2-(diethylaminoethyl)-1H-pyrrol [3.4-c] pyridine-1.3(2H)-dione.

4) 7-amino-2-(2-diethylaminoethyl)-6-methyl-1H-pyrrol [3.4-c] pyridine-1.3(2H)-dione.

5) 7-amino-4-chlorine-2-(diethylaminoethyl)-6-methyl-1H-pyrrol [3.4-c] pyridine-1.3(2H)-dione.

6) 7-amino-2-(2-dimethylaminoethyl)-6-methyl-1H-pyrrol [3.4-c] pyridine-1.3(2H)-dione.

7) 7-amino-2-(diysopropylaminoethyl)-6-methyl-1H-pyrrol [3.4-c] pyridine-1.3(2H)-dione.

8) 7-amino-2-(2-pyrrolidine or ethyl)-6-methyl-1H-pyrrol [3.4-c]pyridine-1.3(2H)-dione.

9) 7-amino-2-(2-piperidine ethyl)-6-methyl-1H-pyrrol [3.4-c] pyridine-1.3(2H)-dione.

10) 7-amino-2-(2-morpholine ethyl)-6-methyl-1H-pyrrol [3.4-c] pyridine-1.3(2H)-dione.

11) 7-amino-2- 2-(4-methylpiperazine) ethyl -6-methyl- 1H-pyrrol [3.4-c]pyridine-1.3(2H)-dione.

12) 7-amino-2-(3-diethylaminopropyl)-6-methyl-1H-pyrrol [3.4-c] pyridine-1.3(2H)-dione.

13) 7-amino-4-chlorine-2-(2-dimethylaminoethyl)-6-methyl-1H-pyrrol [3.4-c] pyridine-1.3(2H)-dione.

14) 7-amino-4-chlorine-2-(2-diysopropylaminoethyl)-6-methyl-1H-pyrrol [3.4-c] pyridine-1.3(2H)-dione.

15) 7-amino-4-chlorine-2-(2-pyrrolidineethyl)-6-methyl-1H-pyrrol [3.4-c] pyridine-1.3(2H)-dione.

16) 7-amino-4-chlorine-2-(piperidineethyl)-6-methyl-1H-pyrrol [3. 4-c] pyridine-1.3(2H)-dione.

17) 7-amino-4-chlorine-2-(2-morpholineethyl)-6-methyl-1H-pyrrol [3.4-c] pyridine-1.3(2H)-dione.

18) 7-amino-4-chlorine-2-(3-diethylaminopropyl)-6-methyl-1H-pyrrol [3.4-c] pyridine-1.3(2H)-dione.

19) Pharmaceutical composition comprising at least one of the compounds of the preceding claims.

20) Employment of the compounds according to the preceding claims for the treatment of cataract.

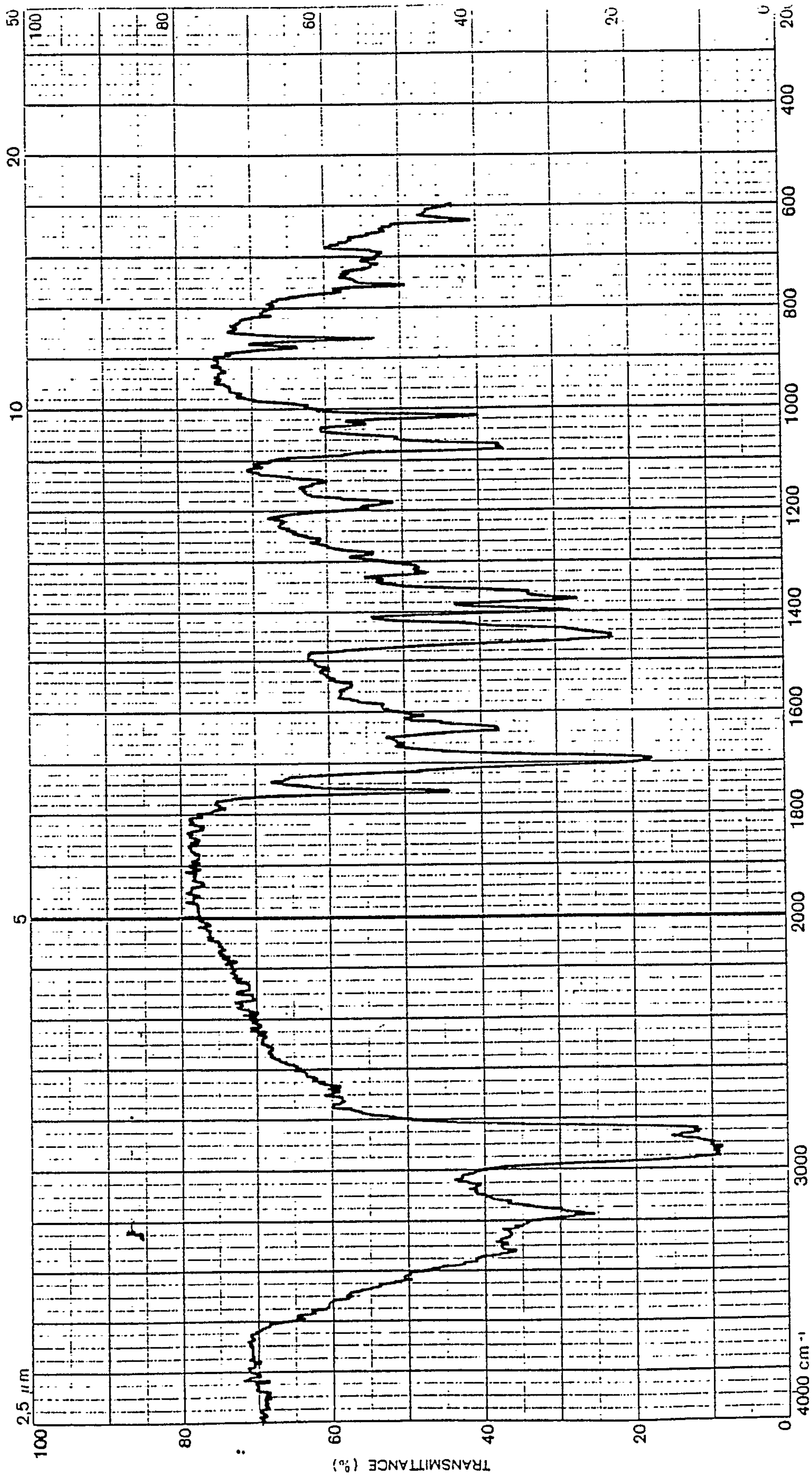


Fig. 1

Fig. 2

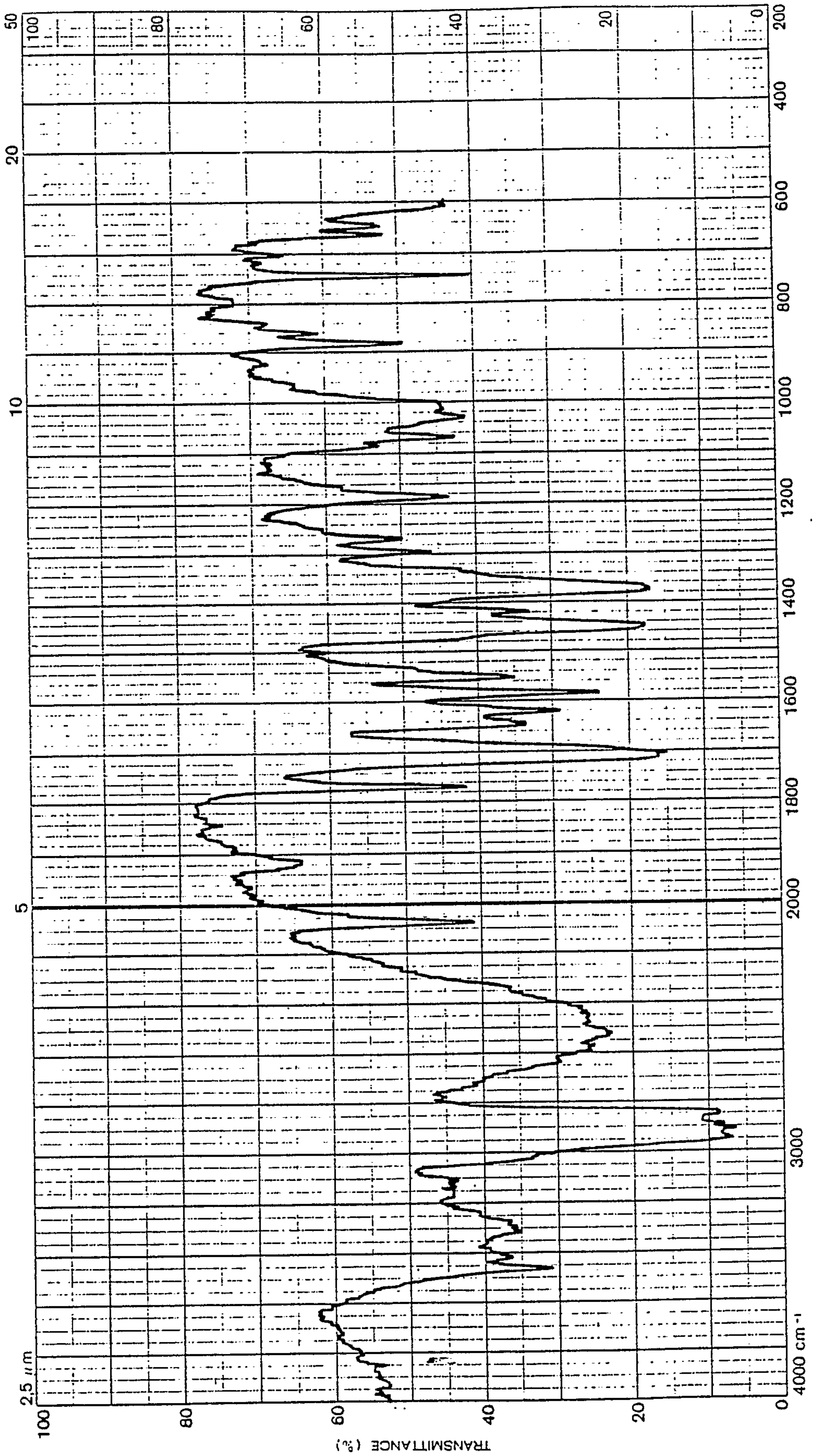
TRANSMITTANCE (%)
100
80
60
40
20
0
4000 cm$^{-1}$
3000
2000
1800
1600
1400
1200
1000
800
600
400
200
2,5 μm
5
10
20
50

Fig 3

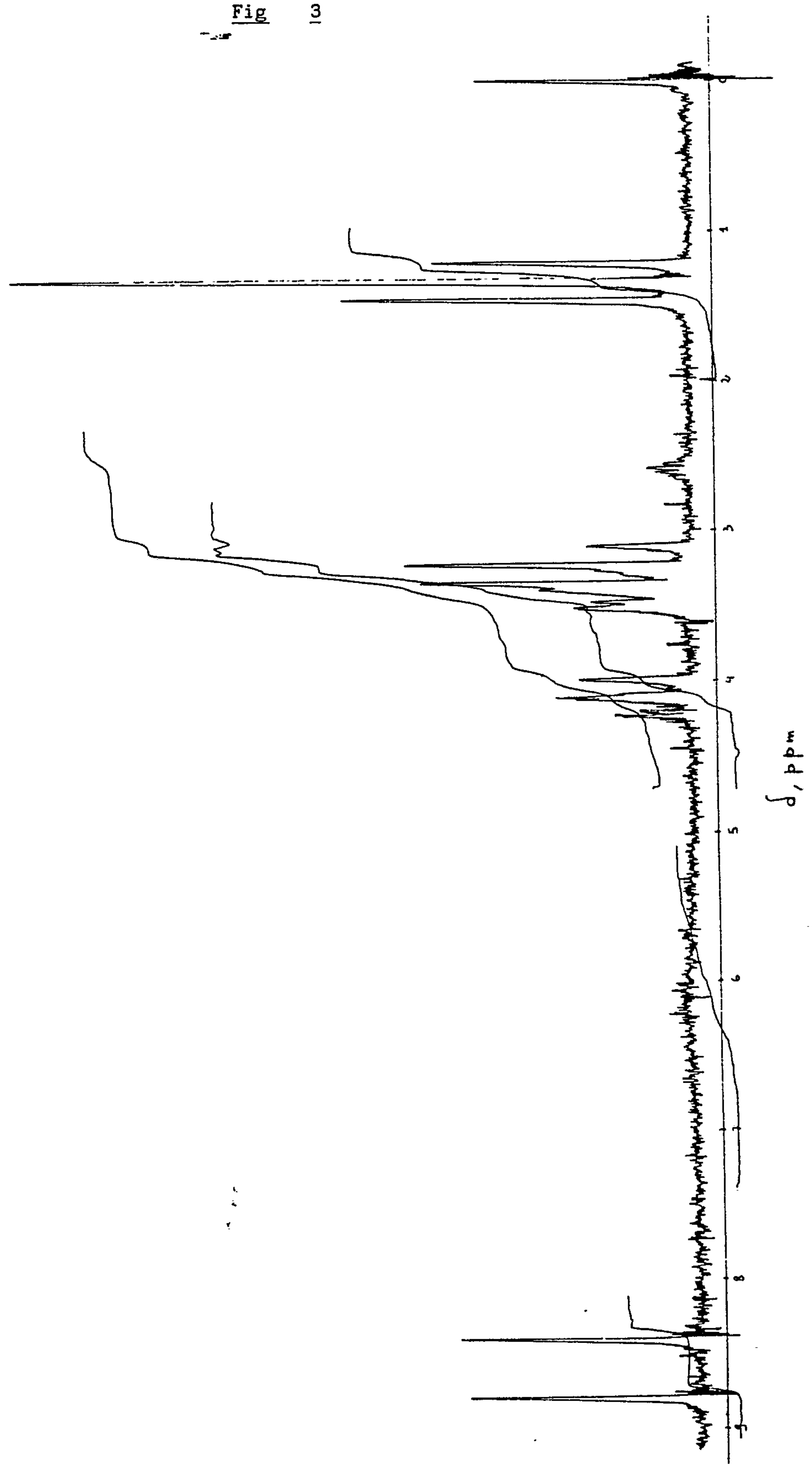

Fig. 4

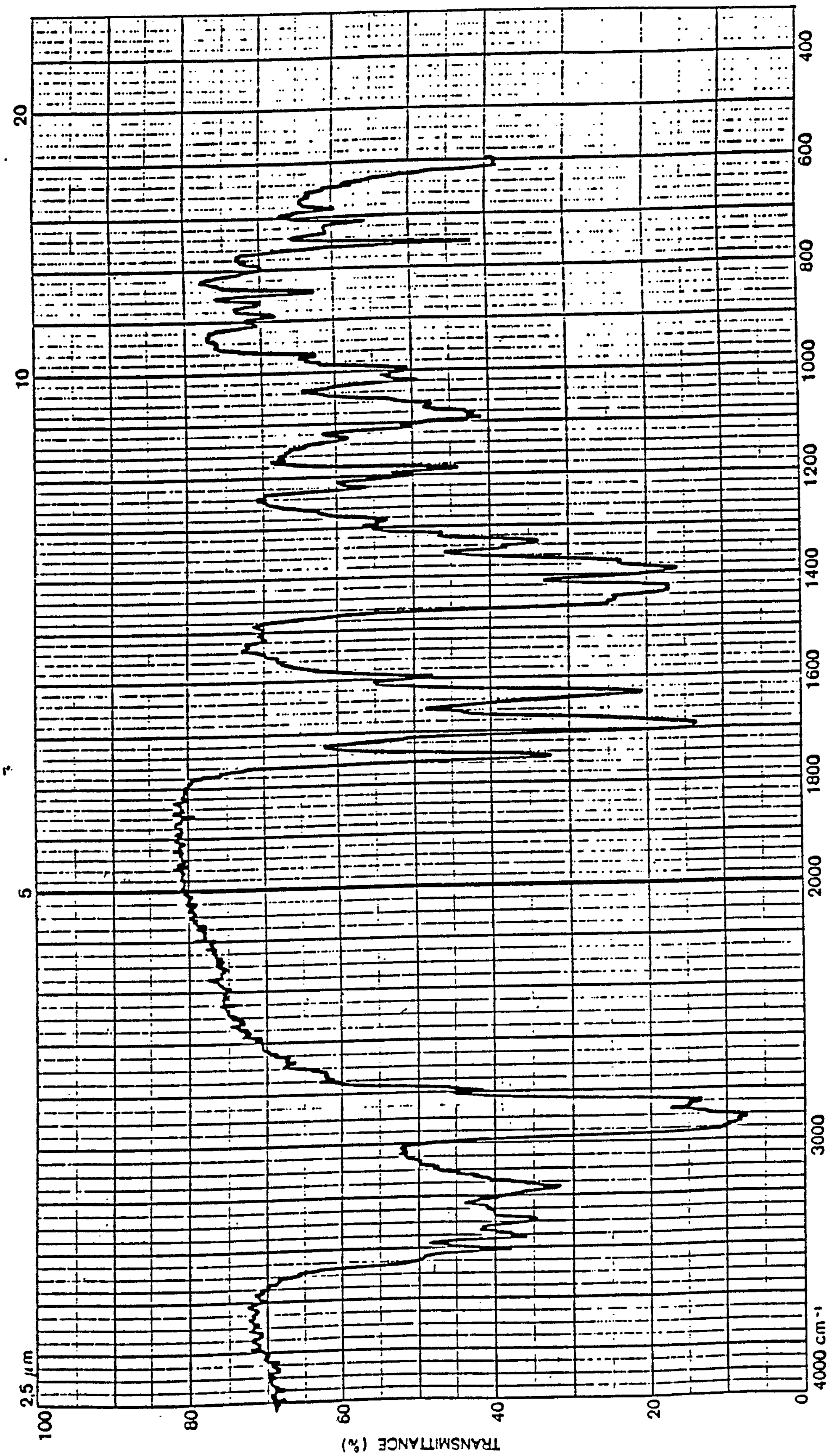
2.5 μm
5
10
20
4000 cm⁻¹
3000
2000
1800
1600
1400
1200
1000
800
600
400
100
80
60
40
20
0
TRANSMITTANCE (%)

Fig 5

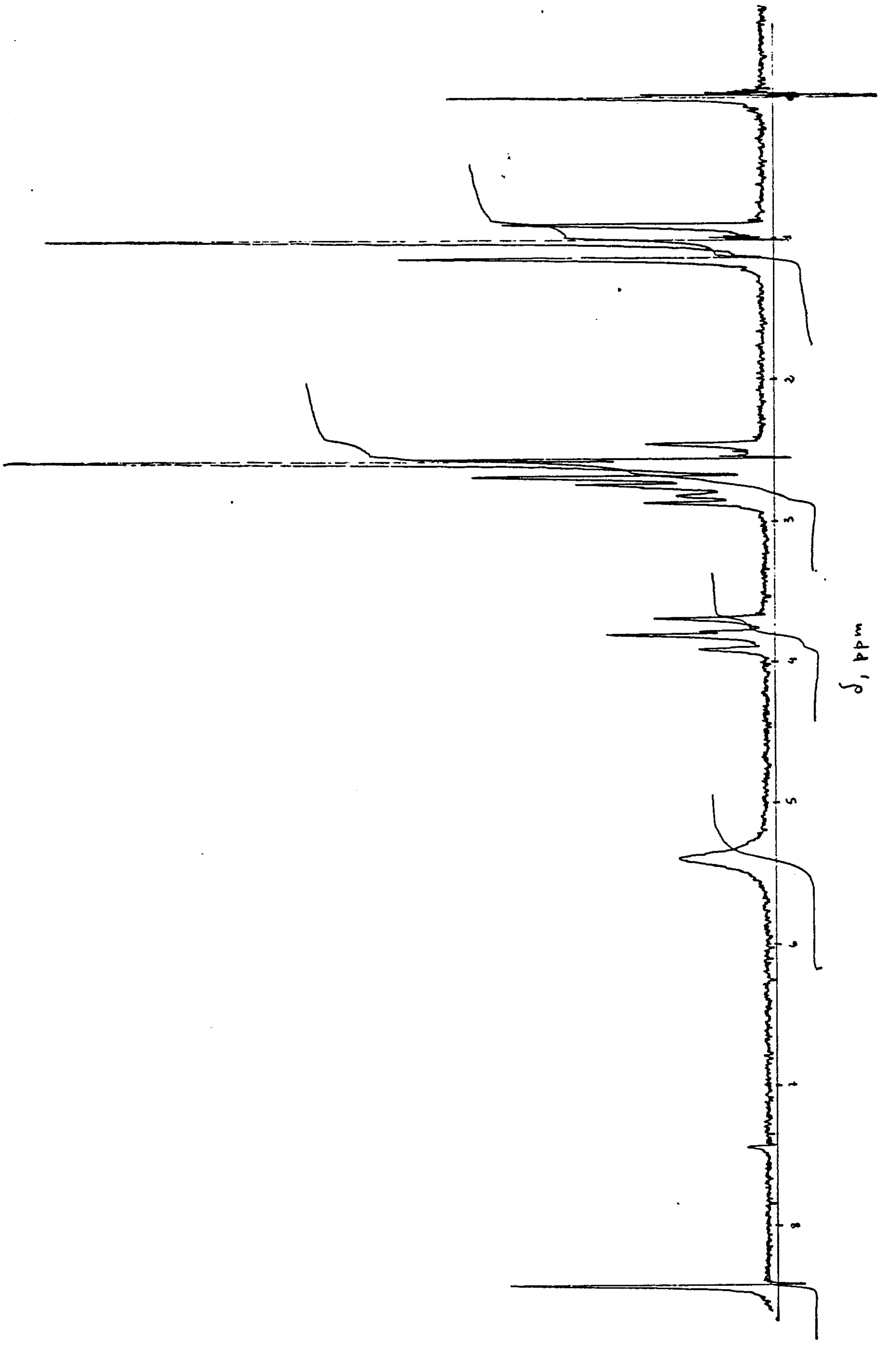

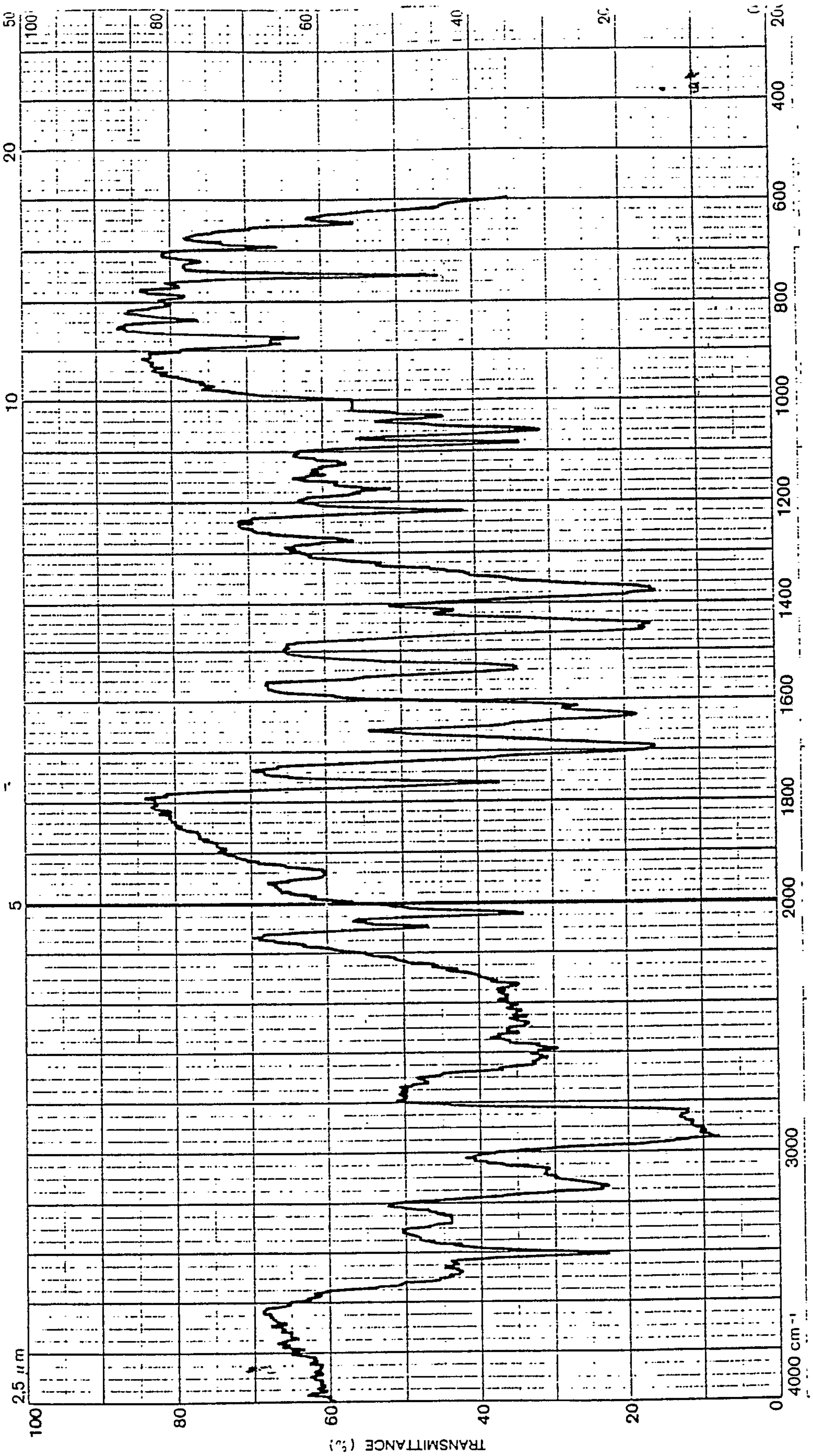
TRANSMITTANCE (%)
100
80
60
40
20
0
2,5 µm
5
10
20
4000 cm⁻¹
3000
2000
1800
1600
1400
1200
1000
800
600
400

Fig. 6

Fig. 7

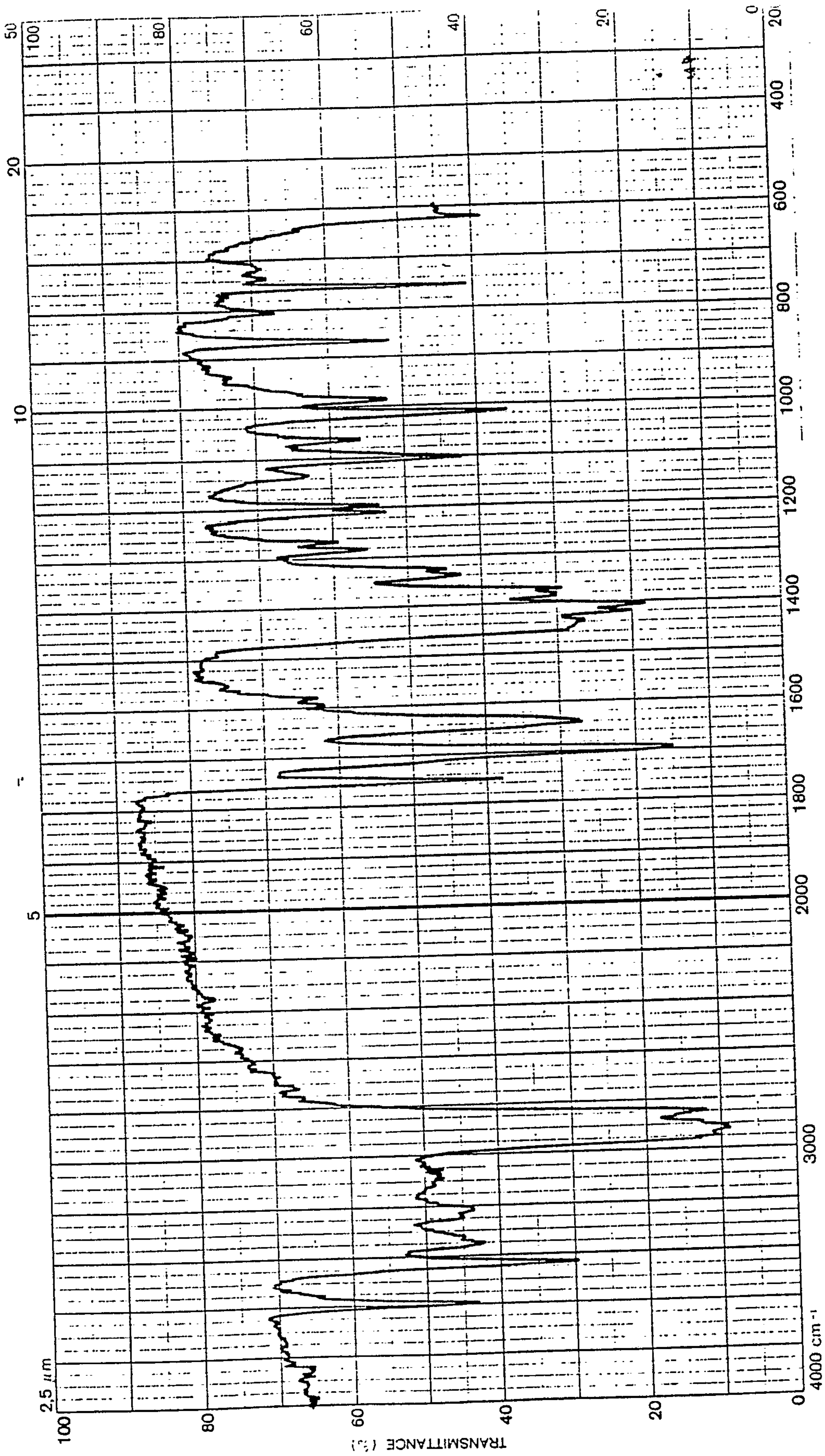
TRANSMITTANCE (%)
100
80
60
40
20
0
2,5 µm
5
10
20
50
4000 cm⁻¹
3000
2000
1800
1600
1400
1200
1000
800
600
400
200

Fig 8

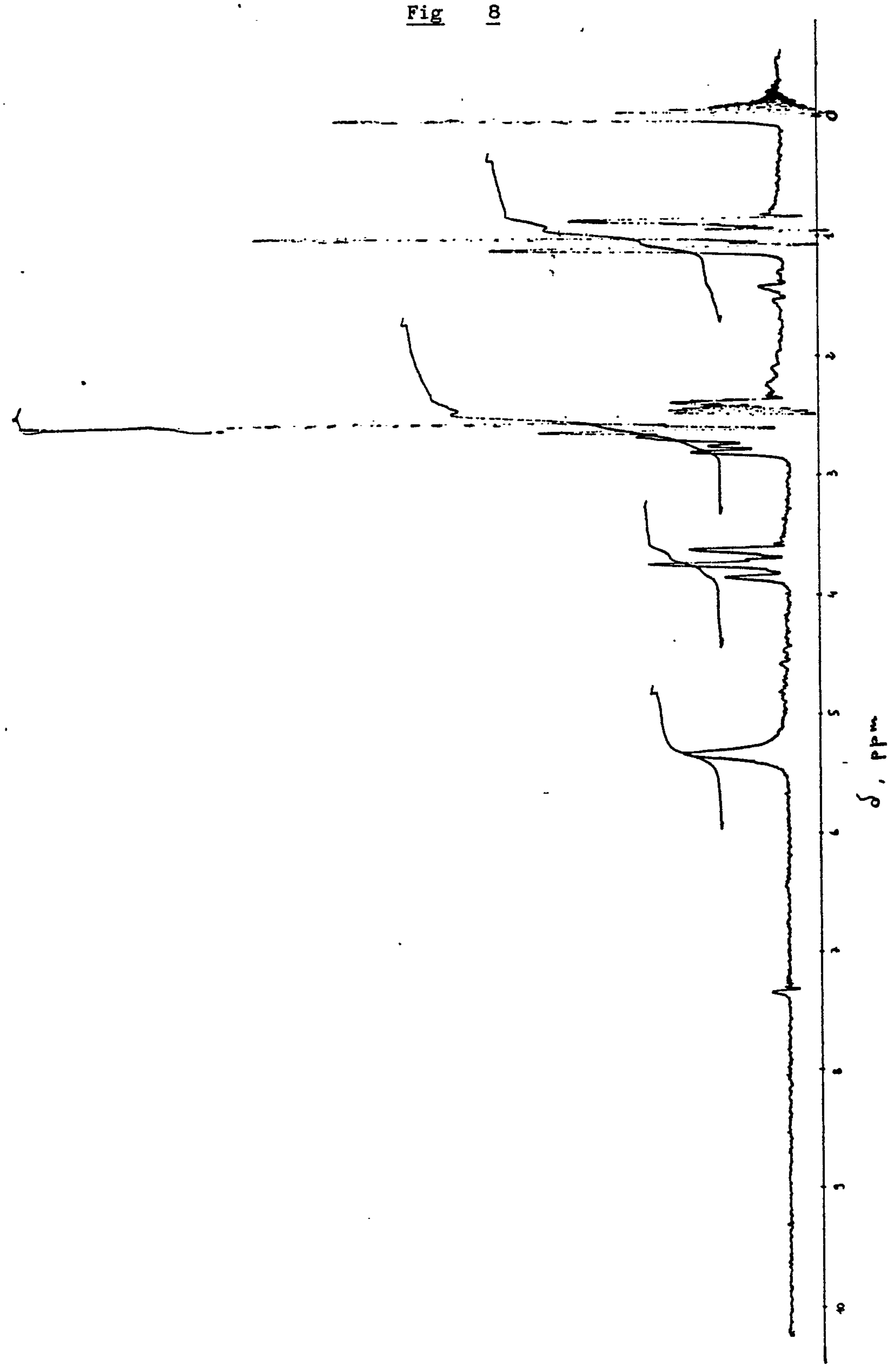

Fig. 9

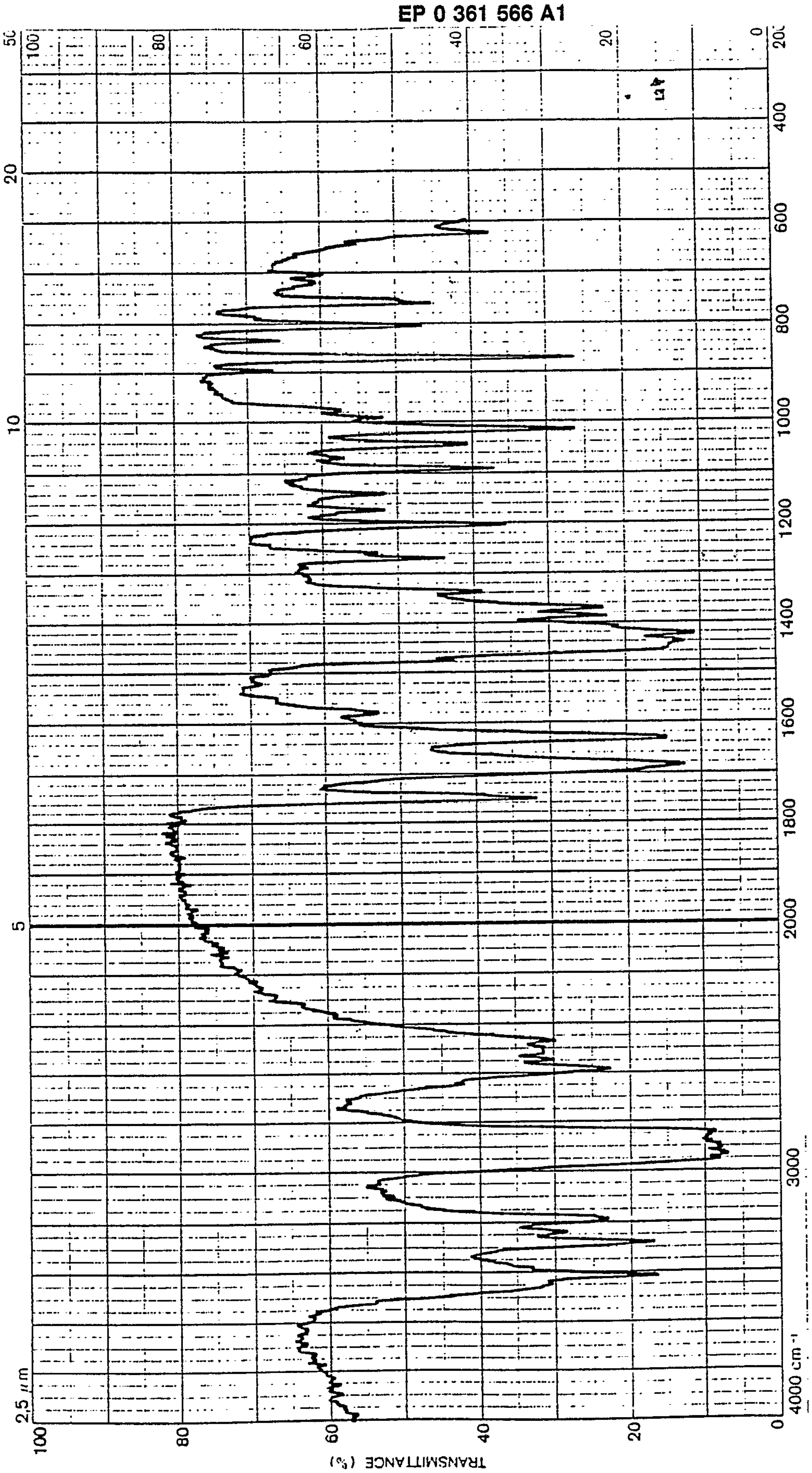
TRANSMITTANCE (%)
2.5 μm
5
10
20
50
4000 cm⁻¹
3000
2000
1800
1600
1400
1200
1000
800
600
400
200
0
20
40
60
80
100

European Patent Office

# PARTIAL EUROPEAN SEARCH REPORT

which under Rule 45 of the European Patent Convention shall be considered, for the purposes of subsequent proceedings, as the European search report

Application number: EP 89202198.1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | DE - A1 - 3 122 635 (BASF) * Claim 1 * | 1 | C 07 D 471/04<br>A 61 K 31/44<br>//(C 07 D 471/04<br>C 07 D 221:00<br>C 07 D 209:00) |
| A | CHEMICAL ABSTRACTS, vol. 91, no. 11, September 10, 1979, Columbus, Ohio, USA S:SHIMADA "7-Amino-2-phenyl-pyrrolo(3,4c)pyridine-1,3-diones" page 767, column 2, Abstract-no. 91 509r & Jpn. Kokai Tokkyo Koho 79 32,476 | 1 | |
| A | CHEMICAL ABSTRACTS, vol. 108, no. 23, June 6, 1988, Columbus, Ohio, USA S.SHIMADA "Reaction of 5-(substituted-amino)oxazoles with N-phenylmaleinimide" page 664, column 1, Abstract-no. 204 522e & J. Heterocycl. Chem. 1987, | 1 | |

TECHNICAL FIELDS SEARCHED (Int. Cl.4)

C 07 D 471/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-19

Claims searched incompletely: -

Claims not searched: 20

Reason for the limitation of the search:

(Article 52(4) EPC; method for the treatment of human or animal body by therapy)

| Place of search | Date of completion of the search | Examiner |
|---|---|---|
| VIENNA | 14-11-1989 | ONDER |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1505.1. 03.82

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| | 24(5) 1237 - 41<br>-- | | |
| A | CHEMICAL ABSTRACTS, vol. 100, no. 15, April 9, 1984, Columbus, Ohio, USA<br>S.SHIMADA et al. "A ono-pot synthesis of 3-aminopyridines"<br>page 571, column 2, Abstract-no. 120 837h<br>& Chem. Pharm. Bull. 1983, 31(10), 3460 - 4<br>-- | 1 | |
| A | CHEMICAL ABSTRACTS, vol. 100, no. 25, June 18, 1984, Columbus, Ohio, USA<br>S.SHIMADA et al. "A convenient synthesis of pyridoxine"<br>page 575, column 2, Abstract-no. 209 594z<br>& Chem. Pharm. Bull. 1984, 32(1), 38 - 43<br>-- | 1 | TECHNICAL FIELDS SEARCHED (Int. Cl.4) |
| A | US - A - 4 379 153 (HITZEL)<br>* Abstract *<br>-- | 1,19 | |
| A | PATENT ABSTRACTS OF JAPAN, unexamined applications, field C, vol. 6, no. 169, September 2, 1982<br>THE PATENT OFFICE JAPANESE GOVERNMENT<br>page 96 C 122<br>* Kokai-no. 57-85 386 (HIROSHI HARA) *<br>---- | 1,19 | |